# EUROPEAN PATENT APPLICATION

(11) **EP 2 233 130 A1**
(43) Date of publication of application: **29.09.2010**
(21) Application number: 09010598.2
(22) Date of filing: 18.08.2009
(51) Int. Cl.: A61K 9/20, A61K 31/554

(54) **A sustained release oral composition of an antipsychotic agent**

(30) Priority: 23.03.2009 IN MU06702009
(71) Applicant: Genepharm (Europe) Trading Limited, 1066 Nicosia (CY)
(72) Inventor: Kumar, Murpani, Deepak, Ashok, New Delhi 110024 (IN); Alexaki, Pandora, 18121 Athens (GR)
(74) Representative: Winkler, Andreas Fritz Ernst

(57) **Abstract**

A sustained release oral composition of an antipsychotic agent comprising antipsychotic agent, λ-carrageenan and one or more pharmaceutically acceptable excipients; wherein the antipsychotic agent is 2-{2-[4-(dibenzo[b,f][1,4]thiazepin-11-yl)piperazino]-ethoxy}ethanol or its pharmaceutically acceptable salt, and a process for the preparation thereof.

## Description

The present invention relates to a sustained release oral pharmaceutical composition of an antipsychotic agent, 2-{2-[4-(dibenzo[b,f][1,4]thiazepin-11-yl)piperazino]-ethoxy}ethanol or quetiapine (INN name) and its pharmaceutically acceptable salt. Antipsychotic agents are normally used to treat psychotropic disorders and other mental/emotional conditions including schizophrenia and acute manic episodes associated with bipolar I disorder.

### Background of the invention

United States Patent No. 4,879,288 (assigned to ICI Americas Inc., referred to herein as '288 patent) discloses 11-[4-[2-(2-hydroxyethoxy)ethyl]-1-piperazinyl}dibenzo[b,f][1,4]thiazepine as a novel antipsychotic drug of the dibenzothiazepine class suitable for various psychotropic disorders and having less side effects. The '288 patent exemplifies immediate release tablets of 11-[4-[2-(2-hydroxyethoxy)ethyl]-1-piperazinyl}dibenzo[b,f][1,4]thiazepine comprising lactose, pregelatinized starch, maize starch, stearic acid, polyvinylpyrrolidone and magnesium stearate.

A sustained release oral dosage form of phenothiazines derivatives is a desired approach in the treatment of psychotropic disorders. The sustained release formulation
(a) avoids frequent administration of the drug while maintaining uniform and constant release rate of the active pharmaceutical ingredient over an extended period of time and
(b) maintains effective plasma drug concentration and controlled release rate of the medicament after a solid oral drug administration.

PCT Publication No. 97/45124 (in the name of Zeneca) discloses a sustained release formulation comprising a gelling agent and quetiapine or a pharmaceutically acceptable salt thereof, together with one or more pharmaceutically acceptable excipients. The gelling agent is hydroxypropyl methyl cellulose and the pharmaceutically acceptable excipient is selected from the group consisting of microcrystalline cellulose, lactose, magnesium stearate, sodium citrate and povidone. Alternate formulations with non-gelling agents may provide sustained release at a steady rate.

PCT Publication No. 01 21179 (in the name of AstraZeneca) discloses a granule formulation comprising quetiapine or a pharmaceutically acceptable salt thereof and a freely or very water-soluble binder. The granules are prepared by using fluid bed technology. The granules are dissolved or suspended in an aqueous medium and then administered to patients with central nervous system disorders.

PCT Publication No. 03/039516 (in the name of Fujisawa) discloses a method for improving the dissolution of a poorly dispersible medicament like quetiapine, which comprises mixing the poorly dispersible medicament with a floating agent and/or a surfactant and granulating the mixture.

PCT Publication No. 2005/041935 (in the name of Alpharma) discloses a sustained release solid dosage formulation comprising a matrix, wherein the matrix comprises a therapeutically effective amount of quetiapine or a pharmaceutically acceptable salt thereof, and a wax material. These formulations may not provide sustained release at a steady rate and drug release may be incomplete due to hydrophobic nature of wax.

We have now surprisingly found that by using lambda carrageenan in the dosage form with the active pharmaceutical ingredient, quetiapine or its pharmaceutically acceptable salt, provides a sustained release dissolution profile of quetiapine can be obtained.

### Object of the invention

The object of the present invention is to provide a sustained release oral composition of an antipsychotic agent, 2-{2-[4-(dibenzo[b,f][1,4]thiazepin-11-yl)piperazino]-ethoxy}ethanol or its pharmaceutically acceptable salt, with the use of lambda carrageenan.

### Summary of the Invention

A sustained release oral composition of an antipsychotic agent comprises an antipsychotic agent, λ-carrageenan and one or more pharmaceutically acceptable excipients; wherein the antipsychotic agent is 2-{2-[4-(dibenzo[b,f][1,4]thiazepin-11-yl)piperazino]-ethoxy}ethanol or its pharmaceutically acceptable salt.

A process for the preparation of a sustained release oral composition of an antipsychotic agent comprises
(i) mixing the antipsychotic agent with λ-carrageenan and one or more pharmaceutically acceptable excipients;
(ii) compacting or granulating the mixture of (i) followed by milling;
(iii) drying and optionally compressing;
wherein the antipsychotic agent is 2-{2-[4-(dibenzo[b,fl[1,4]thiazepin-11-yl)piperazino]-ethoxy}ethanol or its pharmaceutically acceptable salt.

### Detailed Description of the Invention

One embodiment of the present invention is related to a sustained release oral composition of 2-{2-[4-(dibenzo[b,f][1,4]thiazepin-11-yl)piperazino]-ethoxy}ethanol or its pharmaceutically acceptable salt with lambda carrageenan and one or more pharmaceutically acceptable excipients.

The use of λ-carrageenan reportedly provides distinct advantages such as
(a) zero order or steady release of 2-{2-[4-(dibenzo[b,f][1,4]thiazepin-11-yl)piperazino]-ethoxy} ethanol
(b) provides a steady release of 2-{2-[4-(dibenzo[b,f][1,4]thiazepin-11-yl)piperazino]-ethoxy}ethanol in most organic solvents as λ-carrageenan is insoluble therein. Therefore by consumption of alcohol, the pharmacokinetic profile is not affected.
(c) As it is pH independent, it provides a constant rate of release in the GIT.

The USPNF 23 describes carrageenan as hydrocolloid obtained by extraction with water or aqueous alkali from some members of the class Rhodophyceae (red seaweed). It consists mainly of potassium, sodium, calcium magnesium and ammonium sulfate esters of galactose and 3,6- anhydrogalactose copolymers. These hexoses are alternatively linked at the α-1,3 and β-1,4 sites in the polymer.

The carrageenans are divided into three families according to the position of sulfate groups and the presence of anhydrogalactose.

λ-Carrageenan (lambda - carrageenan) is a nongelling polymer containing about 35% ester sulfate by weight and no 3,6-anhydrogalactose.

τ-Carrageenan (iota - carrageenan) is a gelling polymer containing about 32% ester sulfate by weight and approximately 30% 3,6-anhydrogalactose.

κ-Carrageenan (kappa - carrageenan) is a strongly gelling polymer which has a helical tertiary structure that allows gelling. It contains 25% ester sulfate by weight and approximately 34% 3,6-anhydrogalactose.

Among the three carrageenans we have found that the lambda (λ-) carrageenan is the only nongelling polymer.

λ-Carrageenan when formulated with 2-{2-[4-(dibenzo[b,f][1,4]thiazepin-11-yl)piperazino]-ethoxy}ethanol or its pharmaceutically acceptable salt and optionally other pharmaceutical excipient(s) provides sustained or extended release of 2-{2-[4-(dibenzo[b,f][1,4]thiazepin-11-yl)piperazino]-ethoxyl ethanol.

The weight ratio of 2-{2-[4-(dibenzo[b,f][1,4]thiazepin-11-yl)piperazino]-ethoxy}ethanol or its pharmaceutically acceptable salt to λ-carrageenan may range from 1:0.1 to 1:10.

The sustained release oral composition of the present invention comprises one or more pharmaceutical excipient(s). The pharmaceutical excipient used in the oral composition of the present invention must be compatible with 2-{2-[4-(dibenzo[b,f][1,4]thiazepin-11-yl)piperazino]-ethoxy}ethanol or its pharmaceutically acceptable salt. The pharmaceutical excipient may be selected from diluents, binders, lubricants, disintegrants, flavoring agents, coloring agents, stabilizers, surfactants, glidants, plasticizers, preservatives and sweeteners.

Diluents may be selected from calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol, sorbitol, starch, pregelatinized starch, sucrose, talc, xylitol, maltose, maltodextrin, maltitol.

Binders may be selected from acacia, alginic acid, carbomer, carboxymethylcellulose calcium, carbomethylcellulose sodium, microcrystalline cellulose, powdered cellulose, ethyl cellulose, gelatin liquid glucose, guar gum, hydroxyethyl cellulose, hydroxypropyl cellulose, hydroxypropylmethyl cellulose, maltodextrin, methylcellulose, polydextrose, polyethtylene oxide, povidone, sodium alginate, starch paste, pregelatinized starch, sucrose, tragacanth, low-substituted hydroxypropyl cellulose, glucose, sorbitol. Suitable fillers are preferably selected from atleast one of starch derivatives, such as corn starch, potato starch or rice starch. Polysaccharides such as dextrins, maltodextrins, dextrates, microcrystalline cellulose, powdered cellulose, mixture of microcrystalline cellulose and guar gum, coprocessed blends of microcrystalline cellulose; and polyhydric alcohols, such as xylitol and sorbitol.

Disintegrants may be selected from alginic acid, carbon dioxide, carboxymethylcellulose calcium, carboxymethylcellulose sodium, microcrystalline cellulose, powdered cellulose, croscarmelose sodium, crospovidone, sodium docusate, gaur gum, hydroxypropyl cellulose, methylcellulose, polacrilin potassium , poloxamer, povidone, sodium alginate, sodium glycine carbonate, sodium laulyl sulfate, sodium starch glycolate, starch, pregelatinized starch, low-substituted hydroxypropyl cellulose.

Glidants may be selected from calcium silicate, powdered cellulose, starch, talc, colloidal silicon dioxide.

Lubricants may be selected from magnesium stearate, stearic acid, sodium stearyl fumarate, magnesium lauryl sulphate, talc, polyethylene glycol, and glyceryl behenate.

Suitable sweeteners may be selected from sugars such as sucrose, lactose and glucose; cyclamate and salts thereof; saccharin and salts thereof; and aspartame.

Flavouring agents may be selected from natural or synthetic flavours such as strawberry flavour, wild cherry flavour, green apple flavour, spearmint flavour and peppermint flavour.

In a further optional embodiment of the invention, the sustained release oral composition further comprises a coating. The coating material may be selected from materials known to a person skilled in the art.

Another embodiment of the present invention is related to a sustained release oral composition of 2-{2-[4-(dibenzo[b,f][1,4]thiazepin-11-yl)piperazino]-ethoxy}ethanol or its pharmaceutically acceptable salt with lambda carrageenan and one or more pharmaceutically acceptable excipients wherein the dissolution profile of the oral composition releases 10 to 40% in 2 hrs and greater than 60% in more than 12 hrs of 2-{2-[4-(dibenzo[b,f][1,4]thiazepin-11-yl)piperazino]-ethoxy}ethanol.

Yet another embodiment of the present invention is related to the process for the preparation of sustained release oral composition of 2-{2-[4-(dibenzo[b,f][1,4]thiazepin-11-yl)piperazino]-ethoxy}ethanol or its pharmaceutically acceptable salt comprising 2-{2-[4-(dibenzo[b,f][1,4]thiazepin-11-yl)piperazino]-ethoxy}ethanol or its pharmaceutically acceptable salt, lambda carrageenan and one or more pharmaceutical excipient(s). 2-{2-[4-(dibenzo[b,f][1,4]thiazepin-11-yl)piperazino]-ethoxy}ethanol, lambda carrageenan and one or more pharmaceutical excipient(s) are mixed, compacted or granulated, milled, dried and optionally compressed.

The dosage form of the present invention may be prepared using conventional techniques employed in the art for mixing, compaction, granulation, milling, drying and compressing.

The oral composition may be selected from sprinkle granules or powder for reconstitution in a suspension, tablet, soluble tablet, rapidly disintegrating tablet, orally disintegrating tablet, rapidly disintegrating film, orally disintegrating powder for capsules, suspension or sachets, effervescent tablet, a chewable tablet, water dispersible tablet, orodispersible tablet, a chewing gum and suspension.

The oral composition of the present invention was subjected to dissolution method (0.1N hydrochloric acid 750 ml, paddle 50 rpm. After 2 hrs addition of 250 ml of phosphate buffer to obtain pH 6.2)

The following examples illustrate preferred embodiments in accordance with the present invention without limiting the scope of the invention.

### EXAMPLES

| Examples | 1 | 2 | 3 | 4 | 5 | 6 | 7 |
|---|---|---|---|---|---|---|---|
| Ingredients | mg/tab | mg/tab | mg/tab | mg/tab | mg/tab | mg/tab | mg/tab |
| Quetiapine hemifumarate | 230.5 | 230.5 | 230.5 | 230.5 | 230.5 | 230.5 | 230.5 |
| Viscarin 209 (Lambda-Carrageenan) | 250 | 250 | 250 | 250 | 200 | 200 | 150 |
| Microcrystalline cellulose | 64.5 | --- | 64.5 | 113.5 | 113.5 | 99 | 99 |
| Lactose SD | --- | 64.5 | --- | --- | --- | --- | --- |
| Glyceryl behenate | --- | --- | --- | --- | --- | --- | 50 |
| Povidone K30 | --- | --- | --- | --- | --- | 15 | 15 |
| Mg Stearate | 5 | 5 | 5 | 6 | 6 | 5.5 | 5.5 |
| | | | | | | | |
| Total weight | 550 | 550 | 550 | 600 | 550 | 550 | 550 |
| | | | | | | | |
| | | | | | | | |
| Process for the preparation of granules before compression into tablets | Compaction and sizing through 24 mesh | Compaction and sizing through 24 mesh | Wet granulation with purified water | Wet granulation with purified water | Wet granulation with purified water | Wet granulation with PVP binder solution | Wet granulation with PVP binder solution |

### Example 8 Dissolution profile of oral composition of the present invention

| Dissolution profile | Example 3 | Example 4 | Example 5 | | | Example 6 |
|---|---|---|---|---|---|---|
| TIME(h) | 50rpm | 50rpm | 50rpm | 50rpm | 100 rpm | 100 rpm |
| 1 | 10.6 | 9.2 | 14.0 | 14.5 | 17.4 | 15.3 |
| 2 | 19.4 | 17.4 | 26.5 | 27.6 | 31.3 | 27.1 |
| 4 | 31.2 | 30.8 | 39.0 | 39.8 | 47.5 | 42.1 |
| 6 | 41.2 | 41.7 | 49.0 | 48.6 | 60.4 | 54.9 |
| 8 | 50.6 | 51.1 | 56.7 | 56.7 | 71.4 | 66.0 |
| 12 | 67.3 | 65.4 | 69.9 | 70.6 | 89.9 | 84.1 |
| 16 | 78.2 | 77.0 | 80.7 | 80.6 | 99.4 | 94.0 |

### Example 9 Dissolution profile of tablets of prior art

| TIME(hrs) | SEROQUEL 200mg NN0049 (CANADA) | SEROQUEL 200mg FL076 (GREECE) | |
|---|---|---|---|
| | 50rpm | 50rpm | 100rpm |
| 1 | 18.7 | 17.8 | 21.7 |
| 2 | 33.0 | 30.8 | 38.0 |
| 4 | 39.8 | 38.7 | 49.2 |
| 6 | 46.5 | 43.5 | 56.5 |
| 8 | 53.9 | 49.3 | 65.3 |
| 12 | 66.8 | 60.9 | 79.9 |
| 16 | 82.0 | 70.3 | 89.9 |

## Claims

1. A sustained release oral composition of an antipsychotic agent comprising antipsychotic agent, λ-carrageenan and one or more pharmaceutically acceptable excipients; wherein the antipsychotic agent is 2-{2-[4-(dibenzo[b,f][1,4]thiazepin-11-yl)piperazino]-ethoxy}ethanol or its pharmaceutically acceptable salt.

2. The sustained release oral composition according to claim 1 wherein the oral composition is selected from sprinkle granules or powder for reconstitution in a suspension, tablet, soluble tablet, rapidly disintegrating tablet, orally disintegrating tablet, rapidly disintegrating film, orally disintegrating powder for capsules, suspension or sachets, effervescent tablet, a chewable tablet, water dispersible tablet, orodispersible tablet, a chewing gum and suspension.

3. The sustained release oral composition according to claim 1 or 2 wherein the pharmaceutical excipient is selected from diluents, binders, lubricants, disintegrants, flavoring agents, coloring agents, stabilizers, surfactants, glidants, plasticizers, preservatives and sweeteners.

4. The sustained release oral composition according to claim 3 wherein the pharmaceutical excipient is selected from lactose, povidone, glyceryl behenate, microcrystalline cellulose and magnesium stearate.

5. The sustained release oral composition according to any of claims 1 to 4 wherein the ratio of 2-{2-[4-(dibenzo[b,f][1,4]thiazepin-11-yl)piperazino]-ethoxy}ethanol or its pharmaceutically acceptable salt to λ-carrageenan is 1:0.1 to 1:10.

6. The sustained release oral composition according to any of claims 2 to 4 further comprising a coating.

7. The sustained release oral composition according to any of claims 1 to 6 wherein the dissolution profile of the oral composition releases 10 to 40% in 2 hrs and greater than 60% in more than 12 hrs of 2-{2-[4-(dibenzo[b,f][1,4]thiazepin-11-yl)piperazino]-ethoxy}ethanol.

8. A process for the preparation of a sustained release oral composition of an antipsychotic agent comprising
(i) mixing the antipsychotic agent with λ-carrageenan and one or more pharmaceutically acceptable excipients;
(ii) compacting or granulating the mixture of (i) followed by milling;
(iii) drying and optionally compressing;
wherein the antipsychotic agent is 2-{2-[4-(dibenzo[b,f][1,4]thiazepin-11-yl)piperazino]-ethoxy}ethanol or its pharmaceutically acceptable salt.
